Europäisches Patentamt

European Patent Office

Office européen des brevets

(11) Veröffentlichungsnummer : **0 172 478
B1**

(12) **EUROPÄISCHE PATENTSCHRIFT**

(45) Veröffentlichungstag der Patentschrift :
23.11.89

(21) Anmeldenummer : 85109767.5

(22) Anmeldetag : 02.08.85

(51) Int. Cl.⁴ : **C 12 M   3/00**

(54) Verfahren und Vorrichtung zur blasenfreien Begasung von Flüssigkeiten, insbesondere von Kulturmedien zur Vermehrung von Gewebekulturen.

(30) Priorität : 03.08.84 DE 3428758
22.08.84 DE 3430924·

(43) Veröffentlichungstag der Anmeldung :
26.02.86 Patentblatt 86/09

(45) Bekanntmachung des Hinweises auf die Patenterteilung : 23.11.89 Patentblatt 89/47

(84) Benannte Vertragsstaaten :
AT BE CH DE FR GB IT LI NL SE

(56) Entgegenhaltungen :
WO--A--85 /021 95
FR--A-- 2 201 903
FR--A-- 2 393 535
GB--A-- 1 480 406
GB--A-- 2 059 436
GB--A-- 2 093 730

(73) Patentinhaber : Gesellschaft für Biotechnologische Forschung mbH (GBF)
Mascheroder Weg 1
D-3300 Braunschweig-Stöckheim (DE)

(72) Erfinder : Lehmann, Jürgen, Dr.-Ing.
Mascheroder Weg 1
D-3300 Braunschweig (DE)

(74) Vertreter : Boeters, Hans Dietrich, Dr. et al
Boeters, Bauer & Partner Thomas-Wimmer-Ring 14
D-8000 München 22 (DE)

## Beschreibung

Die Erfindung betrifft ein Verfahren und eine Vorrichtung zur blasenfreien Begasung von Flüssigkeiten, insbesondere von Kulturmedien insbesondere zur Vermehrung von Gewebekulturen.

Die Begasung von Flüssigkeiten dient dem Eintrag und der Desorption von Gasen. Insbesondere bei der Vermehrung von tierischen Zellen in Kulturmedien stellt die ausreichende Sauerstoffversorgung und Kohlendioxid-Entsorgung ein Problem dar, wenn Zellzahlen von mehr als $10^6$ Zellen pro ml Kulturmedium erreicht werden sollen. Da mit der sogenannten Oberflächenbegasung nur ein geringes Flüssigkeitsvolumen begast werden kann, ist man zu der sogenannten submersen Begasung übergegangen. Hierbei können zwar die geforderten Gastransportraten mit Luft oder reinem Sauerstoff erreicht werden, wobei es aber zu einer unerwünschten Schaumbildung an der Oberfläche des Mediums kommt. In diesem Schaum tritt häufig eine Flotation der Zellen oder auch von Mikrocarriern und damit eine Leistungsverminderung der Kultur auf.

Zur Lösung dieser Probleme ist eine Methode bekannt geworden, bei der ein Gasaustausch über eine eingetauchte Membranfläche erfolgt. Hierbei wird die Begasung mit geschlossenen oder offenporigen Membranen durchgeführt, die in der durch einen Rührer bewegten Flüssigkeit angeordnet werden. Dies ist z. B. durch die PCT/SE 84/00382 bekannt geworden, die vorschlägt, einen Membrankorb einzusetzen, der aus die Diffusion von Sauerstoff zulassendem Material besteht, und der in eine translatorische Bewegung, z. B. eine Kreispendelbewegung, versetzt werden kann. Eine derartige Diffusionsmembran nennt auch die GB-A-2 059 436.

Es ist auch bereits bekannt geworden, offenporiges Membranmaterial einzusetzen, da es hier nicht zum Anwachsen von Zellen auf der Membranoberfläche kommt. Da hierbei die Gefahr besteht, daß es zu Gasblasenbildung und damit zu Schaumbildung kommt, werden die bekannten offenporigen Membranhohlfasern vor dem Gebrauch benetzbar gemacht und mit Reaktionsmedium gefüllt (siehe DE-A 31 07 874 = GB-A-2 093 730). In diesem Fall findet der Gasaustausch mit der Flüssigkeit an der inneren Oberfläche der Hohlfaser statt, und es besteht die Gefahr, daß das Medium durch die Poren nach innen durchbricht.

Der Erfindung liegt die Aufgabe zugrunde, ein Verfahren zur blasenfreien Begasung von Flüssigkeiten, insbesondere von Kulturmedien, insbesondere zur Vermehrung von Gewebekulturen, und eine dafür geeignete Vorrichtung der eingangs genannten Art zu schaffen, mit dem bzw. mit der einfach und wirkungsvoll eine möglichst gleichmäßige Verteilung von Gaseintrag und Gasdesorption möglich ist, ohne daß Zellen oder Carrier durch Schaumbildung beschädigt werden.

Der Erfindung liegt ferner die Aufgabe zugrunde, für das genannte Verfahren und die Vorrichtung offenporige Membranhohlfäden zur Verfügung zu stellen, die hydrophob sind und deren Poren nicht mit Reaktionsmedium gefüllt sind.

Diese Aufgabe wird verfahrensseitig durch die im Kennzeichen des Anspruch 1 genannten Merkmale gelöst.

Vorrichtungsseitig sind zur Lösung dieser Aufgabe die im Kennzeichen des Anspruchs 5 genannten Merkmale vorgesehen.

Die erfindungsgemäßen Membranhohlfäden sind hydrophob, so daß kein Wasser in die Membran eindringen kann. Dadurch wird die Trennfläche Gas/Flüssigkeit auf die äußere Oberfläche verlagert. Die Leistungsfähigkeit dieser Membranfäden übersteigt die der bekannten mikroporösen Fäden ganz wesentlich ; bei geeigneter Wahl der Druckverhältnisse läßt sich eine bisher nicht bekannte Sauerstoffübertragungsrate (siehe unten) erzielen, ohne daß es zum Ausbrechen von Glasblasen in das Medium kommt. Ein weiterer Vorteil ist, daß die Fäden nicht vorbehandelt werden müssen, bevor sie eingesetzt werden.

Bevorzugte Merkmale, die das erfindungsgemäße Verfahren und die erfindunsgemäße Vorrichtung vorteilhaft weiterbilden, sind in den entsprechend nachgeordneten Ansprüchen genannt.

Als für diese Erfindung besonders geeignete Hohlfäden haben sich Fäden aus hydrophobem Polypropylen erwiesen, beispielsweise nach dem Accurelverfahren hergestellte Hohlfäden.

Durch die erfindungsgemäße Ausgestaltung des Verfahrens und der Vorrichtung wird eine gleichmäßige Flüssigkeitsbewegung an allen Stellen der Membran bzw. des Membrankorbs und damit eine hohe Grenzflächenerneuerungsgeschwindigkeit infolge der Bewegung des Membrankorbs garantiert. Dabei finden sowohl der Gaseintrag als auch die Gasdesorption quasi homogen verteilt über das ganze Flüssigkeitsvolumen statt, ohne daß zusätzliche Mischorgane erforderlich sind. Die Gaszufuhr und die Gasabfuhr kann in vorteilhafter Weise ohne bewegte Abdichtungen über eine einfache Kopplung mit Hilfe von flexiblen Schläuchen erfolgen, wobei die Schläuche zugleich zur Aufhängung des Membrankorbs dienen können. Der röhrenförmig gestaltete Membrankorb kann in vorteilhafter Weise in seiner Ausbildung leicht den besonderen Verhältnissen in Zellkulturen, z. B. Schleimbildung, Zellagglomeratbildung, angepaßt werden. Dies kann in günstiger Weise beispielsweise durch Änderung der Zahl und der Durchmesser der Membran-Hohlfäden problemlos vorgenommen werden.

In vorteilhafter Weiterbildung der Erfindung kann zur Vereinfachung der Bewegungsaufprägung vorgesehen sein, daß der Membrankorb ohne Eigendrehung bewegt wird.

Die translatorische Bewegung des Membrankorbs, die vorzugsweise ohne Eigendrehung des Membrankorbs vorgesehen ist, kann beispielsweise über die Membrankorbaufhängung eingeleitet werden. Dabei ist es vorteilhaft, wenn der Membrankorb einen Magnetkern aufweist, dem von einer außerhalb des zu begasenden Mediums vorgesehenen Magnetanordnung die Pendel-, Drehpendel- und/oder Schwingungsbewegung vermittelt wird. Die Magnetanordnung kann dabei selbst bewegt werden oder eine entsprechend bewegende Krafteinleitung vornehmen.

Insbesondere für größere Flüssigkeitsmengen ist es alternativ vorteilhaft, wenn ein entsprechend maßstäblich vergrößerter Membrankorb über Gelenkglieder aufgehängt und mit Hilfe eines im Bereich der Symmetrieachse des Membrankorbs arbeitenden Exzenters bewegt wird. Hierbei kann der Exzenter vorzugsweise in eine am Membrankorb befestigte Führungsscheibe eingreifen, wobei es besonders günstig ist, wenn die Symmetrieachse der Führungsscheibe mit der des Membrankorbs zusammenfällt und wenn die Führungsscheibe mittels eines Spannringträgers an wenigstens einem geschlitzten Spannring befestigt ist, der mit den Membranhohlfäden den Membrankorb bildet.

Die Begasungsrate kann vorzugsweise durch Änderung der Kreispendelfrequenz und/oder des Radius der Kreisbahn oder alternativ durch Änderung der Gaskonzentration und/oder des Druckes des in den Membrankorb einströmenden Gases gesteuert werden.

Weitere Einzelheiten, Merkmale und Vorteile der Erfindung sind dem anschließenden Beschreibungsteil zu entnehmen, in dem die Erfindung anhand von zwei schematisch dargestellten Beispielen einer erfindungsgemäßen Vorrichtung und Untersuchungsbeispielen näher erläutert wird.

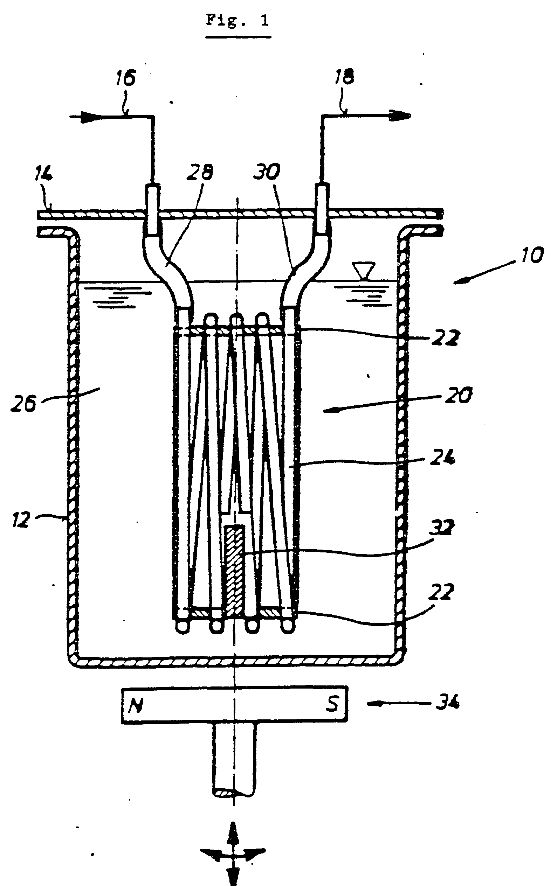

Der in der Figur 1 dargestellte schematische Aufbau einer erfindungsgemäßen Vorrichtung 10 besteht aus einem unmagnetischen Behälter 12, auf dem ein Deckel 14 angeordnet ist, der gegebenenfalls in nicht dargestellter Weise befestigt und abgedichtet sein kann. Durch den Deckel 14 sind eine Gaseintrittsleitung 16 und eine Gasaustrittsleitung 18 geführt.

In dem Behälter 12 ist ein Membrankorb 20 angeordnet, der einen hohlzylinderförmigen Membranträger 22 besitzt, auf den Membranhohlfäden 24 schraubenartig aufgewickelt sind. Der Membrankorb 20 ist in eine sich in dem Behälter 12 befindende Flüssigkeit 26 vollständig eingetaucht und wird an seinem oberen Ende von elastischen Schläuchen 28 und 30 flexibel gehalten. Der Schlauch 28 ist mit der Gaseintrittsleitung 16 und einem Ende des aufgewickelten Membranhohlfadens 24 verbunden, während der Schlauch 30 das andere Ende des aufgewickelten Membranhohlfadens 24 mit der Gasaustrittsleitung 18 verbindet.

Im unteren Bereich des Membrankorbs 20 ist an dem Membranträger 22 drehfest ein Magnetkern 32 angebracht, der von einer Magnetanordnung 34 beaufschlagbar ist, die außerhalb des Behälters 12 angeordnet ist. Die Magnetanordnung 34 ist über eine nicht dargestellte Einrichtung linear hin- und herbewegbar, um über den Magnetkern 32 dem Membrankorb 20 eine Pendelbewegung zu vermitteln. Diese lineare Bewegung erfolgt in einer derartigen Richtung, daß der Membrankorb 20 μm eine durch die Aufhängungspunkte laufende Gerade pendelt.

Im unteren Bereich des Membrankorbs 20 ist an dem Membranträger 22 drehfest ein Magnetkern 32 angebracht, der von einer Magnetanordnung 34 beaufschlagbar ist, die außerhalb des Behälters 12 angeordnet ist. Die Magnetanordnung 34 ist über eine nicht dargestellte Einrichtung drehbar, um über den Magnetkern 32 dem Membrankorb 20 eine Kreispendelbewegung zu vermitteln. Bei dieser Kreispendelbewegung bewegt sich der Membrankorb ohne eigene Drehung um seine Symmetrieachse auf einer Kreisbahn.

Alternativ ist es auch möglich, eine lineare Bewegung unter einem bestimmten Winkel zur Symmetrieachse des Membrankorbs einzuleiten, um einer linearen Pendelbewegung des Membrankorbs aufgrund der Elastizität der Schläuche 28 und 30 eine zusätzliche Bewegungskomponente zu verleihen, die zu einer elliptischen bzw. einer kreisförmigen Pendelbewegung führen kann. Es ist darüber hinaus auch möglich, anstelle der entsprechend bewegten Magnetanordnung 34 eine stationäre Elektromagnetanordnung vorzusehen, wobei die gewünschte Pendel- oder Drehpendelbewegung des Membrankorbs durch eine entsprechende Anordnung und Ansteuerung der Elektromagneten erreicht werden kann.

Die Begasungsrate läßt sich durch Änderung der Kreispendelfrequenz und/oder des Radius der Kreisbahn steuern. Bei dem dargestellten Ausführungsbeispiel kann eine entsprechende Änderung des Kreisbahnradius in einfacher Weise dadurch vorgenommen werden, daß in dem Deckel 14 der Abstand zwischen der Gaseintrittsleitung 16 und der Gasaustrittsleitung 18 verändert wird. Beispielsweise kann durch Verkleinerung des genannten Abstandes die Membrankorbauslenkung aus der Ruhelage (d. h. derjenigen Lage, in der dem Membrankorb 20 keine Bewegung aufgeprägt wird und dieser unbewegt in dem Behälter 12 hängt) entsprechend verringert werden.

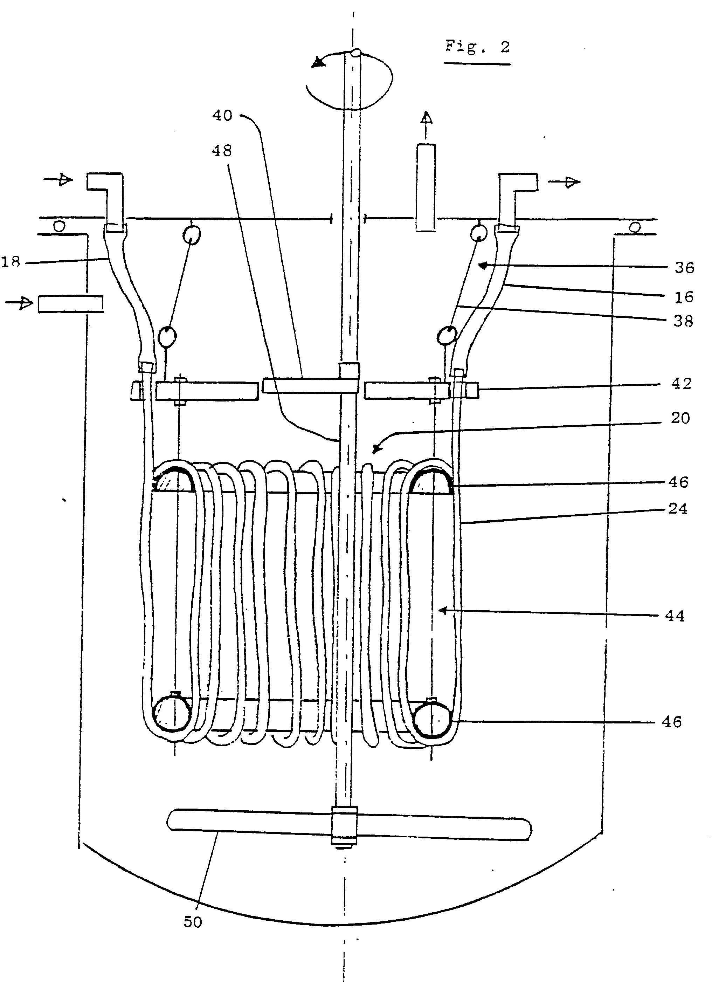

Das in der Figur 1 dargestellte Ausführungsbeispiel ist vor allem für kleinere Flüssigkeitsmengen vorgesehen. Für die blasenfreie Begasung von Flüssigkeitsmengen in der Größenordnung von 30 l und mehr wird gemäß Figur 2 die Aufhängung über die elastischen Schläuche 16, 18 für die Zu- und Abfuhr von Gas vorteilhaft durch eine geeignete Aufhängung 36 mit biegsamen oder schwenkbaren Gelenkgliedern (38) vorgenommen und der maßstäblich vergrößerte Membrankorb 20 mit Hilfe eines Exzenters 40 bewegt. Die Gelenkglieder 38 können beispielsweise aus zwei oder mehr gleichlangen Zugdrähten bestehen, die mit Abstand voneinander an dem Deckel 14 gelenkig angreifen und gelenkig mit einer den Exzenter 40 umgebenden Führungsscheibe 42 verbunden sind, deren Symmetrieachse mit der des Membrankorbs 20 zusammenfällt. Diese Führungsscheibe 42 kann mittels eines Spannringträgers 44 an geschlitzten Spannringen 46 befestigt sein, die mit dem Membranhohlfaden oder den Membranhohlfäden 24 den entsprechend vergrößerten Membrankorb bilden. Zur Aufprägung einer Kreispendelbewegung auf den Membrankorb 20 kann eine in den Behälter 12 eingreifende Antriebswelle 48 vorgesehen sein, die mit dem in seiner Exzentrizität einstellbaren Exzenter 40 an der bzw. in die Führungsscheibe 42 eingreift. Die Antriebswelle 48 tritt dabei vorzugsweise durch den Deckel 14 oder den Boden des Behälters 12 im Bereich der Behältersymmetrieachse ein. Falls die Antriebswelle 48 durch den Deckel 14 des Behälters 12 eingeführt wird, ist die Führungsscheibe 42 vorteilhaft oberhalb des Membrankorbs 20 angeordnet, und die Welle 48 kann durch den hohlzylindrischen Membrankorb 20 hinaus in die untere Hälfte des Behälters 12 verlängert und an ihrem unteren Ende mit einem Rührer 50 versehen sein. Dieser Rührer 50 kann während der ersten Züchtungsstufe einer Kultur in der sogenannten Anzuchtsphase bei einem niedrigen Flüsigkeitspegel und reiner Belüftung über die Oberfläche eine langsame Bewegung der Flüssigkeit vornehmen.

Die zuvor beschriebene Vorrichtung ist auch als Zusatzsystem für herkömmliche Fermenter geeignet und bildet eine ideale Ergänzung für vorhandene Systeme.

Zur Untersuchung der Leistungsfähigkeit der in der Figur 1 schematisch dargestellten Vorrichtung wurde ein Membrankorb 20 hergestellt, auf dem ein Membranhohlfaden 24 aus Accurel[R] Typ PX 190/1/2/8366 mit einem Porenvolumen von 75 % und einer Porengröße von 0,3 $\mu$m aufgewickelt worden war. Die Länge des Membranhohlfadens 24 betrug 121 cm, die äußere Membranoberfläche 98,7 cm$^2$, und der Membranhohlfaden wurde mit reinem Sauerstoff bei einem Überdruck von 30 mm WS von innen begast. Als Membranmaterial ist besonders mikroporöses Polypropylen, beispielsweise Accurel[R], günstig.

Der Membrankorb wurde in 1,3 l destilliertes Wasser eingetaucht und mit einer Auslenkung von 2,5 cm um die Mitte mit 60 Kreispendelungen pro Minute durch einen unter dem Behälter drehbar angeordneten Magneten bewegt.

Messungen an dieser Vorrichtung ergaben, daß bei einer Temperatur von 25 °C eine Sauerstoffübertragungsrate OTR von 56 mg $O_2$/(l · h) ohne Austreten von Sauerstoffblasen aus der Membranoberfläche erreicht werden konnte. Dies entspricht einem spezifischen Sauerstofffluß von $Q_{O2}$ von 0,57 mg $O_2$/(cm$^2$ · h). An der unbewegten Oberfläche wurde an der gleichen Vorrichtung ein Sauerstofffluß $Q_{O2}$ von 0,22 mg $O_2$/(cm$^2$ · h) gemessen.

Die beschriebene Vorrichtung ist beispielsweise für die Züchtung von Fibroblasten, z. B. der humanen diploiden Vorhautzellen (FS-4), geeignet, die einen spezifischen Sauerstoffbedarf $q_{O2}$ von 0,05 mmol $O_2$/(10$^9$ Zellen · h) oder 1,6 mg $O_2$/(10$^9$ Zellen · h) haben. Es wurde berechnet, daß mit dieser Vorrichtung 3,5 · 10$^{10}$ Zellen/l versorgt werden könnten.

Nachfolgend wird die Anwendung des Verfahrens und der Vorrichtung in einem anderen Beispiel beschrieben. Eine Zellkultur mit primären menschlichen Leukozyten mit einer Zellzahl n von 4,5 · 10$^9$ Zellen/l und einem anfänglichen spezifischen Sauerstoffbedarf $q_{O2}$ von 4,3 mg $O_2$/(10$^9$ Zellen · h) wurde bei 37 °C in einem Kulturmedium RPMI 1640 und einigen prozeßbedingten Zusätzen bei 60 Kreispendelungen pro Minute und einem Sauerstoffüberdruck von 30 mm WS behandelt. Dabei wurde eine Sauerstoffübertragungsrate OTR von 38 mg $O_2$/(l · h) bei blasenfreier Begasung erreicht. Dieser Wert übersteigt den notwendigen Wert um 18,5 mg $O_2$/(l · h). Um die Sauerstoffübertragungsrate auf den Bedarf von 19,5 mg $O_2$/(l · h) zu verringern, wurde eine einfache Absenkung der Kreispendelfrequenz vorgenommen.

Eine bedarfsabhängige Steuerung der Sauerstoffversorgung kann auch durch eine Veränderung der Gaskonzentration im einströmenden Gas erreicht werden. Bei der vorstehend beschriebenen Vorrichtung wurde beispielsweise eine Regelung vorgenommen, bei der ein Sollwert von 10 % Luftsättigung derart aufrecht erhalten wurde, das durch die Membran ein kontinuierlicher Luftstrom von 1 Nl/h geleitet wurde, dem im Bedarfsfall reiner Sauerstoff zugemischt wurde. Die Zumischung erfolgte mittels eines durch einen Zweipunkt-Regler gesteuerten Magnetventils, wobei der Istwert der Luftsättigung im Medium mit einer Sauerstoffelektrode gemessen wurde.

Die in der Zellkulturtechnik übliche Einstellung des pH-Wertes über ein Puffer-$CO_2$-Gleichgewicht läßt sich durch Zumischen der notwendigen $CO_2$-Menge in das Eintrittsgas erreichen.

Das erfindungsgemäße Verfahren und die erfindungsgemäße Vorrichtung eignen sich auch in vorteilhafter Weise für ein blasenfreies Begasen von Mikrocarrier-Kulturen.

| | |
|---|---|
| 10 Vorrichtung | apparatus |
| 12 Behälter | vessel |
| 14 Deckel | lid |
| 16 Gaszutrittsleitung | gas inlet line |
| 18 Gasaustrittsleitung | gas outlet line |
| 20 Membrankorb | membrane cage |
| 22 Membranträger | membrane carrier |
| 24 Membranhohlfaden | hollow membrane filament |
| 26 Flüssigkeit | liquid |
| 28 Schlauch | tube |
| 30 Schlauch | tube |
| 32 Magnetkern | magnetic core |

4

| 34 Magnetanordnung | magnet arrangement |
| 36 Aufhängung | suspension system |
| 38 Gelenkglied | articulated member |
| 40 Exzenter | eccentric |
| 42 Führungsscheibe | guide plate |
| 44 Spannringträger | tension ring support |
| 46 Spannring | tension ring |
| 48 Antriebswelle | drive shaft |
| 50 Rührer | stirrer |

**Patentansprüche**

1. Verfahren zur blasenfreien Begasung von Flüssigkeiten, insbesondere von Kulturmedien, insbesondere zur Vermehrung von Gewebekulturen, mit einem Gasaustausch über eine eingetauchte Membranfläche, bei dem ein hohlzylindrischer Membrankorb flexibel in dem zu bewegenden Medium aufgehängt und dem Membrankorb eine translatorische Bewegung (beispielsweise eine Kreispendelbewegung) aufgeprägt wird, dadurch gekennzeichnet, daß man einen Membrankorb verwendet, der aus einem oder mehreren offenporigen Membranhohlfäden gebildet ist, die nicht vor ihrem Einsatz für das Reaktionsmedium benetzbar gemacht worden sind.

2. Verfahren nach Anspruch 1, dadurch gekennzeichnet, daß die Begasungsrate durch Änderung der Kreispendelfrequenz und/oder des Radius der Kreisbahn gesteuert wird.

3. Verfahren nach Anspruch 1, dadurch gekennzeichnet, daß die Begasungsrate durch Änderung der Gaskonzentration des in den Membrankorb einströmenden Gases gesteuert wird.

4. Verfahren nach einem der vorhergehenden Ansprüche, dadurch gekennzeichnet, daß das flexible Aufhängen des Membrankorbs mit Hilfe von elastischen Schläuchen für die Zu- und Abfuhr von Gas vorgenommen wird.

5. Vorrichtung zur blasenfreien Begasung von Flüssigkeiten, insbesondere von Kulturmedien, insbesondere zur Vermehrung von Gewebekulturen, mit einem Gasaustausch über eine eingetauchte Membranfläche und mit einem hohlzylindrischen Membrankorb, der flexibel in dem zu begasenden Medium aufhängbar und translatorisch (beispielsweise kreispendelnd) bewegbar ist, gekennzeichnet durch einen Membrankorb, der aus einem oder mehreren offenporigen Membranhohlfäden gebildet ist, die nicht vor ihrem Einsatz für das Reaktionsmedium benetzbar gemacht worden sind.

6. Vorrichtung nach Anspruch 5, dadurch gekennzeichnet, daß die Begasungsrate durch Änderung der Kreispendelfrequenz und/oder des Radius der Kreisbahn steuerbar ist.

7. Vorrichtung nach Anspruch 5, dadurch gekennzeichnet, daß die Begasungsrate durch Steuerung der Gaskonzentration und/oder des Gasdrucks des in den Membrankorb (20) einströmenden Gases änderbar ist.

8. Vorrichtung nach einem der Ansprüche 5 bis 7, dadurch gekennzeichnet, daß der Membrankorb (20) an elastischen Schläuchen (28, 30) für die Zu- und Abfuhr von Gas aufgehängt ist.

**Claims**

1. A process for the bubble-free gassing of liquids, especially of culture media, especially for propagating tissue cultures, with a gas exchange across an immersed membrane sheet, in which a hollow cylindrical membrane cage is resiliently suspended in the medium to be moved, and a translatory movement (for example a circular pendulum movement) is imparted to the membrane cage, characterised in that there is used a membrane cage that is formed by one or more open-pored hollow membrane filaments, that have not been made wettable before being used for the reaction medium.

2. A process according to claim 1, characterised in that the gassing rate is controlled by altering the circular pendulum frequency and/or the radius of the circular path.

3. A process according to claim 1, characterised in that the gassing rate is controlled by altering the concentration of the gas flowing into the membrane cage.

4. A process according to any one of the preceding claims, characterised in that the membrane cage is resiliently suspended by means of resilient tubes for supplying and discharging the gas.

5. Apparatus for the bubble-free gassing of liquids, especially of culture media, especially for propagating tissue cultures, with a gas exchange across an immersed membrane sheet and with a hollow cylindrical membrane cage which can be resiliently suspended in the medium to be gassed and can be moved in a translatory movement (for example a circular pendulum movement), characterised by a membrane cage that is formed by one or more open-pored hollow membrane filaments, that have not been made wettable before being used for the reaction medium.

6. Apparatus according to claim 5, characterised in that the gassing rate can be controlled by altering the circular pendulum frequency and/or the radius of the circular path.

7. Apparatus according to claim 5, characterised in that the gassing rate can be changed by

## EP 0 172 478 B1

controlling the concentration and/or the pressure of the gas flowing into the membrane cage (20).

8. Apparatus according to any one of claims 5 to 7, characterised in that the membrane cage (20) is suspended on resilient tubes (28, 30) for supplying and discharging gas.

**Revendications**

1. Procédé d'aération, exempt de bulles, de liquides, notamment de milieux de culture, notamment pour un développement de cultures de tissus, avec échange de gaz par l'intermédiaire d'une surface de membrane immergée, selon lequel un panier à membrane de forme cylindrique creuse est suspendu de façon flexible dans le milieu à déplacer et un mouvement de translation (par exemple un mouvement pendulaire circulaire) est produit pour le panier à membrane, caractérisé en ce qu'on utilise un panier à membrane qui est constitué d'un ou plusieurs filaments creux de membrane à pores ouverts, qui n'ont pas été rendus mouillables par le milieu de réaction avant leur utilisation.

2. Procédé selon la revendication 1, caractérisé en ce que la capacité d'aération est commandée par modification de la fréquence pendulaire circulaire et/ou du rayon de la trajectoire circulaire.

3. Procédé selon la revendication 1, caractérisé en ce que la capacité d'aération est commandée par modification de la concentration du gaz pénétrant dans le panier à membrane.

4. Procédé selon une des revendications précédentes, caractérisé en ce que la suspension flexible du panier à membrane est effectuée à l'aide de tuyaux élastiques servant à l'entrée et à la sortie de gaz.

5. Appareil pour l'aération, exempte de bulles, de liquides, notamment de milieux de culture, en particulier pour le développement de cultures de tissus, avec échange de gaz par l'intermédiaire d'une surface de membrane immergée et au moyen d'un panier à membrane de forme cylindrique creuse, qui peut être suspendu de façon flexible dans le milieu à aérer et qui est déplaçable par translation (par exemple par un mouvement pendulaire circulaire), caractérisé par un panier à membrane qui est constitué d'un ou plusieurs filaments creux de membrane à pores ouverts, qui n'ont pas été rendus mouillables par le milieu de réaction avant leur utilisation.

6. Appareil selon la revendication 5, caractérisé en ce que la capacité d'aération peut être commandée par modification de la fréquence pendulaire circulaire et/ou du rayon de la trajectoire circulaire.

7. Appareil selon la revendication 5, caractérisé en ce que la capacité d'aération est modifiable par commande de la concentration et/ou de la pression du gaz pénétrant dans le panier à membrane (20).

8. Appareil selon une des revendications 5 à 7, caractérisé en ce que le panier à membrane (20) est suspendu à des tuyaux élastiques (28, 30) servant à l'entrée et à la sortie du gaz.

Fig. 1

Fig. 2